(19) Europäisches Patentamt
European Patent Office
Office européen des brevets

(11) **EP 4 275 676 B1**

(12) **EUROPEAN PATENT SPECIFICATION**

(45) Date of publication and mention
of the grant of the patent:
**13.08.2025 Bulletin 2025/33**

(21) Application number: **22913275.8**

(22) Date of filing: **01.07.2022**

(51) International Patent Classification (IPC):
*A61K 9/127* (2025.01)     *A61K 31/427* (2006.01)
*A61P 35/00* (2006.01)

(52) Cooperative Patent Classification (CPC):
**A61K 9/1271; A61K 9/1272; A61K 31/427;
A61K 45/06; A61P 35/00**

(86) International application number:
**PCT/CN2022/103468**

(87) International publication number:
**WO 2023/123986 (06.07.2023 Gazette 2023/27)**

(54) **UTIDELONE LIPOSOME COMPOSITION, AND PREPARATION METHOD THEREFOR AND USE THEREOF**

UTIDLONLIPOSOMZUSAMMENSETZUNG UND HERSTELLUNGSVERFAHREN DAFÜR UND
VERWENDUNG DAVON

COMPOSITION DE LIPOSOME D'UTIDÉLONE, PROCÉDÉ DE PRÉPARATION ASSOCIÉ ET
UTILISATION ASSOCIÉE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **31.12.2021 CN 202111672446**

(43) Date of publication of application:
**15.11.2023 Bulletin 2023/46**

(73) Proprietor: **CHENGDU BIOSTAR
PHARMACEUTICALS, LTD.
Sichuan 611730 (CN)**

(72) Inventors:
• **TANG, Li**
**Beijing 101111 (CN)**
• **ZHANG, Chuan**
**Chengdu, Sichuan 611730 (CN)**
• **XU, Jing**
**Chengdu, Sichuan 611730 (CN)**
• **ZHANG, Cheng**
**Chengdu, Sichuan 611730 (CN)**
• **QIU, Rongguo**
**Beijing 101111 (CN)**

(74) Representative: **Høiberg P/S
Adelgade 12
1304 Copenhagen K (DK)**

(56) References cited:
WO-A1-2021/180111     WO-A1-2021/204188
WO-A1-2021/204188     CN-A- 1 813 721
CN-A- 1 813 721     CN-A- 109 863 165
CN-A- 113 402 509     US-A1- 2005 042 275
US-A1- 2009 191 264

• SCHERZINGER-LAUDE K. ET AL.: "Treatment of
neuroblastoma and rhabdomyosarcoma using
RGD-modified liposomal formulations of
patupilone (EPO906)", INTERNATIONAL
JOURNAL OF NANOMEDICINE, 1 June 2013
(2013-06-01), New Zealand, pages 2197,
XP093158585, ISSN: 1178-2013, DOI: 10.2147/
IJN.S44025

Remarks:
The file contains technical information submitted
after the application was filed and not included in this
specification

## Description

### Technical Field

The present invention belongs to the field of pharmaceutical technology, and more specifically to a liposome composition comprising Utidelone (or a salt thereof), a phospholipid, and optionally a sterol, as well as to the preparation method and use thereof.

### Background

Utidelone is a class of epothilone derivatives belonging to macrolides and secondary metabolites produced by the genetically modified *Sorangium Cellulosum.* Studies have shown that epothilones have the same pharmacological mechanism as paclitaxel, which exerts anti-tumor effect by inhibiting the depolymerization of tubulin. The chemical name of Utidelone is: 4,8-dihydroxy-5,5,7,9,13-pentamethyl-16-[1-methyl-2-(2-methyl-thiazole-4-yl) -vinyl]-hexadecoxetane-13-en-2,6-one lactone.

Utidelone is easily soluble in ethanol, methanol, ethyl acetate, and chloroform, but insoluble in water, and has a saturated solubility in water of less than 1 μg/ml. The clinical formulations comprise a large amount of Polyoxyl (35) castor oil and organic solvents, which render a possibility of causing serious allergic reactions. Before clinical application, patients need to be given preventive anti-allergic treatments, and have poor drug compliance.

Patents US20090191264A and CN100409846C describe liposomes prepared from epothilone B. However, the examples described in the specification of US20090191264A use natural phosphatidylcholines or formulas without adding cholesterol. Due to the formulas, their stability *in vivo* and *in vitro* may not be enough to achieve the function of sustained release; while in CN100409846C, the specification describes that the weight ratio of epothilone B is only 0.1% -2%. Patent EP2286795A1 describes cationic liposomes prepared from epothilones. However, the direct application of cationic lipids to human body has a disadvantage of having significant side effects.

Utidelone has a chemical structure which is different from those of the epothilone derivatives described in the above documents, and changes in the chemical structure of the drug often cause significant changes in its physical and chemical properties, thus affecting the interaction between the drug and the excipients, which makes it difficult for Utidelone to be prepared into liposome compositions. As shown in Comparative Example 1 of the present application, the Utidelone liposome as prepared has a poor stability, and the drug-containing solution precipitates quickly during or after the preparation process. A large proportion of the drug is difficult to pass through the sterilization filter, and the drug loading capacity is very low, making the process not feasible.

### Summary of the Invention

Thus, one of the technical problems to be solved by the present invention is to provide a stable Utidelone liposome.

Further, a technical problem to be solved by the present invention is to provide a stable Utidelone liposome having a high drug loading capacity.

Further, a technical problem to be solved by the present invention is to provide a method for preparing a stable Utidelone liposome, which can efficiently encapsulate Utidelone stably in the liposome.

In order to solve the above technical problems, the inventors creatively used the special physical and chemical properties of different types of phospholipids, selected a phospholipid or phospholipid combination suitable for Utidelone, and prepared one or more stable Utidelone liposomes.

In one aspect, the present invention provides a liposome composition, wherein the liposome composition comprises Utidelone and a phospholipid. The present invention also provides a liposome composition, wherein the liposome composition comprises Utidelone, a phospholipid and a sterol. In the liposome composition of the present invention, Utidelone is entrapped in the liposome.

In one or more embodiments, the phospholipid is one or more selected from the group consisting of a pegylated phospholipid, an anionic phospholipid, a cationic phospholipid and a zwitterionic phospholipid.

In one or more embodiments, the phospholipid comprises or consists of a zwitterionic phospholipid.

In one or more embodiments, the phospholipid comprises or consists of a zwitterionic phospholipid in combination with a pegylated phospholipid, an anionic phospholipid and/or a cationic phospholipid.

In one or more embodiments, the phospholipid comprises or consists of a zwitterionic phospholipid in combination with a pegylated phospholipid. In one or more embodiments, the phospholipid comprises or consists of a zwitterionic phospholipid in combination with an anionic phospholipid. In one or more embodiments, the phospholipid comprises or consists of a zwitterionic phospholipid in combination with a cationic phospholipid. In one or more embodiments, the phospholipid comprises or consists of a zwitterionic phospholipid in combination with a pegylated phospholipid and an anionic phospholipid. In one or more embodiments, the phospholipid comprises or consists of a zwitterionic phospholipid in combination with a pegylated phospholipid and a cationic phospholipid.

In one or more embodiments, the zwitterionic phospholipid is one of more selected from the group consisting of egg phosphatidylcholine (EPC), egg phosphatidylserine (EPS), phosphatidylethanolamine (EPE), soybean phosphatidylserine (SPS), soybean phosphatidylethanolamine (SPE), hydrogenated egg phosphatidylcholine (HEPC), hydrogenated egg phosphatidylserine (HEPS), hydrogenated egg phosphatidylethanolamine (HEPE), hydrogenated soybean phosphatidylcholine (HSPC), hydrogenated soybean phosphatidylserine (HSPS), hydrogenated soybean phosphatidylethanolamine (HSPE), dipalmitoyl phosphatidylcholine (DPPC), 1-palmitoyl-2-myristoylphosphatidylcholine (PMPC), 1-myristoyl-2-palmitoylphosphatidylcholine (MPPC), dioleoyl phosphatidylcholine (DOPC), dimyristoyl phosphatidylcholine (DMPC), distearoyl phosphatidylcholine (DSPC), 1-palmitoyl-2-stearoylphosphatidylcholine (PSPC), 1,2-diarachidoyl-sn-glycero-3-phosphatidylcholine (DBPC), 1-stearoyl-2-palmitoyl phosphatidylcholine (SPPC), 1,2-dierucoyl-sn-glycero-3-phosphocholine (DEPC), palmitoyl oleoyl phosphatidylcholine (POPC), dilauroyl phosphatidylcholine (DLPC), palmitoyl stearoyl phosphatidylcholine (PSPC), lysophosphatidylcholine (LPC), dilinoleoyl phosphatidylcholine (DLPC), distearoyl phosphatidylethanolamine (DSPE), dimyristoyl phosphatidylethanolamine (DMPE), dipalmitoyl phosphatidylethanolamine (DPPE), dioleoyl phosphatidylethanolamine (dioleylphosphatidylethanolamine, DOPE), and palmitoyl oleoyl phosphatidylethanolamine (POPE).

In one or more preferred embodiments, the zwitterionic phospholipid is an unsaturated zwitterionic phospholipid. Examples of the unsaturated zwitterionic phospholipid include, but are not limited to, egg phosphatidylcholine (EPC), egg phosphatidylserine (EPS), phosphatidylethanolamine (EPE), soybean phosphatidylserine (SPS), soybean phosphatidylethanolamine (SPE), dioleoyl phosphatidylcholine (DOPC), 1,2-dierucoyl-sn-glycero-3-phosphocholine (DEPC), palmitoyl oleoyl phosphatidylcholine (POPC), dioleoyl phosphatidylethanolamine (DOPE), and palmitoyl oleoyl phosphatidylethanolamine (POPE).

In one or more preferred embodiments, the zwitterionic phospholipid is a synthetic zwitterionic phospholipid. Examples of the synthetic zwitterionic phospholipid include, but are not limited to, DSPC and DOPC.

More preferably, in one or more embodiments, the zwitterionic phospholipid is a synthetic unsaturated zwitterionic phospholipid. Examples of the synthetic unsaturated zwitterionic phospholipid include, but are not limited to, DOPC.

In one or more preferred embodiments, the zwitterionic phospholipid is one or more selected from the group consisting of egg phosphatidylcholine (EPC), hydrogenated soybean phosphatidylcholine (HSPC), dioleoyl phosphatidylcholine (DOPC), and distearoyl phosphatidylcholine (DSPC).

In one or more preferred embodiments, the zwitterionic phospholipid is one or more selected from the group consisting of egg phosphatidylcholine (EPC), dioleoyl phosphatidylcholine (DOPC), and distearoyl phosphatidylcholine (DSPC).

In one or more preferred embodiments, the zwitterionic phospholipid is one or both selected from the group consisting of egg phosphatidylcholine (EPC), and dioleoyl phosphatidylcholine (DOPC).

More preferably, in one or more embodiments, the zwitterionic phospholipid is dioleoyl phosphatidylcholine (DOPC).

In one or more embodiments, the pegylated phospholipid is one or more selected from the group consisting of distearoyl phosphatidylethanolamine-polyethylene glycol (DSPE-PEG), distearoyl phosphatidylethanolamine-methoxypolyethylene glycol (DSPE-MPEG), dimyristoyl phosphatidylethanolamine-polyethylene glycol (DMPE-PEG), dipalmitoylglycero succinate polyethylene glycol (DPGS -PEG), cholesteryl-pegylated phospholipid and ceramido pegylated phospholipid.

In one or more preferred embodiments, the pegylated phospholipid is one or both selected from the group consisting of distearoyl phosphatidylethanolamine-polyethylene glycol (DSPE-PEG) and distearoyl phosphatidylethanolamine-methoxypolyethylene glycol (DSPE-MPEG).

More preferably, in one or more embodiments, the pegylated phospholipid is distearoyl phosphatidylethanolamine-methoxypolyethylene glycol (DSPE-MPEG).

In one or more embodiments, the anionic phospholipid is one or more selected from the group consisting of di(hexadecyl)phosphate (DhP), phosphatidylinositol, phosphatidylserine, phosphatidylglycerol, lysophosphatidylglycerol (lysylphosphatidylglycerol, LPG), phosphatidylethanolamine, phosphatidic acid, cardiolipin, and cholesteryl hemisuccinate.

In one or more embodiments, the phosphatidylserine is one or more selected from the group consisting of dimyristoyl phosphatidylserine, dipalmitoyl phosphatidylserine, and distearoyl phosphatidylserine.

In one or more preferred embodiments, the anionic phospholipid is phosphatidylglycerol.

In one or more embodiments, the phosphatidylglycerol is one or more selected from the group consisting of dimyristoyl phosphatidylglycerol (DMPG), dipalmitoyl phosphatidylglycerol (DPPG), distearoyl phosphatidylglycerol (DSPG), dioleoyl phosphatidylglycerol (DOPG), dilauroyl phosphatidylglycerol (DLPG), egg phosphatidylglycerol (EPG), egg phosphatidylinositol (EPI), soybean phosphatidylglycerol (SPG), soybean phosphatidylinositol (SPI), hydrogenated egg phosphatidylglycerol (HEPG), hydrogenated soybean phosphatidylglycerol (HSPG), hydrogenated egg phosphatidylinositol (HEPI), palmitoyl stearoyl phosphatidylglycerol (PSPG), and hydrogenated soybean phosphatidylinositol (HSPI).

In one or more preferred embodiments, the phosphatidylglycerol is distearoyl phosphatidylglycerol (DSPG).

In one or more embodiments, the cationic phospholipid is one or more selected from the group consisting of N-[1-(2,3-dioleoyloxy)propyl]-N,N,N-trimethylammonium (DOTAP), N-[1-(2,3-dimyristoyl)propyl]-N,N,N-trimethylammonium (DMTAP), N-[1-(2,3-dipalmitoyl)propyl]-N,N,N-trimethylammonium (DPTAP), N-[1-(2,3-distearoyl)propyl]-N,N,N-trimethylammonium (DSTAP), dimethyl di(octadecyl)ammonium bromide (DDAB), 1,2-diacyloxy-3-trimethylammoniumpropane, N-[1-(2,3-dioleoyloxy)propyl]-N,N-dimethylamine (DODAP), 1,2-diacyloxy-3-dimethylammoniumpropane, N-[1-(2,3-dioleoyloxy)propyl]-N,N,N-trimethylammonium chloride (DOTMA), 1,2-dialkyloxy-3-dimethylammoniumpropane, di(octadecyl)amidoglycyl spermine (DOGS), 3-[N-(N',N'-dimethylaminoethane)carbamoyl]|cholesterol (DC-Chol), 2,3-dioleoyl-N-(2-(sperminecarboxamido)ethyl)-N,N-dimethyl-1-propanaminium trifluoroacetate (DOSPA), β-alanyl cholesterol, cetyltrimethylammonium bromide (CTAB), di C14-amidine, N-*tert*-butyl-N'-tetradecyl-3-tetradecylaminopropionamide (N-tert-butyl-N'-tetradecyl-3-tetradecylaminopropionamidine), N-(α-trimethylammonioacetyl)di(dodecyl)-d-glutamate chloride (TMAG), di(tetradecanoyl)-N-(trimethylammonioacetyl)diethanolamine chloride, 1,3-dioleoyloxy-2-(6-carboxy-spermyl)-propionamide (DOSPER) and N,N,N',N'-tetramethyl-N'-bis(2-hydroxyethyl)-2,3-dioleoyloxy-1,4-butanediammonium iodide, 1-[2-(acyloxy)ethyl] 2-alkyl(alkenyl)-3-(2-hydroxyethyl)-imidazolinium chloride derivatives such as 1-[2-(9(Z)-octadecenoyloxy)ethyl]-2-(8(Z)-heptadecenyl-3-(2-hydroxyethyl)-imidazolinium chloride (DOTIM), 1-[2-(hexadecanoyloxy)ethyl]-2-pentadecyl-3-(2-hydroxyethyl)imidazolinium chloride (DPTIM), 1,2-dioleoyl-3-dimethyl-hydroxyethylammonium bromide (DORI), 1,2-dioleoyloxypropyl-3-dimethyl-hydroxyethylammonium bromide (DORIE), 1,2-dioleoyloxypropyl-3-dimethyl-hydroxypropylammonium bromide (DORIC-HP), 1,2-dioleyloxypropyl-3-dimethyl-hydroxyethylammonium bromide (DORIE-HB), 1,2-dioleoyloxypropyl-3-dimethyl-hydroxypentylammonium bromide (DORIE-Hpe), 1,2-dimyristyloxypropyl-3-dimethyl-hydroxyethylammonium bromide (DMRIE), 1,2-dipalmitoyloxypropyl-3-dimethyl-hydroxyethylammonium bromide (DPRIE), 1,2-didecyloxypropyl-3-dimethyl-hydroxyethylammonium bromide (DMRIE), 1,2-distearyloxy-N,N-dimethyl-3-aminopropane (DSDMA), 1,2-dioleoyloxy-N,N-dimethyl-3-aminopropionic acid (DODMA), 1,2-dilinoleyoxy-N,N-dimethyl-3-aminopropane (DLinDMA), 1,2-dilinolenyloxy-N,N-dimethyl-3-aminopropane (DLenDMA).

In one or more embodiments, the phospholipid comprises or consists of an unsaturated zwitterionic phospholipid in combination with an anionic phospholipid. In one or more embodiments, the phospholipid comprises or consists of a synthetic zwitterionic phospholipid in combination with an anionic phospholipid. In one or more preferred embodiments, the phospholipid comprises or consists of a synthetic unsaturated zwitterionic phospholipid in combination with an anionic phospholipid. More preferably, in one or more embodiments, the phospholipid comprises or consists of dioleoyl phosphatidylcholine (DOPC) in combination with distearoyl phosphatidylglycerol (DSPG).

In one or more embodiments, the phospholipid comprises or consists of an unsaturated zwitterionic phospholipid in combination with a pegylated phospholipid. In one or more embodiments, the phospholipid comprises or consists of a synthetic zwitterionic phospholipid in combination with a pegylated phospholipid. In one or more preferred embodiments, the phospholipid comprises or consists of a synthetic unsaturated zwitterionic phospholipid in combination with a pegylated phospholipid. More preferably, in one or more embodiments, the phospholipid comprises or consists of dioleoyl phosphatidylcholine (DOPC) in combination with distearoyl phosphatidylethanolamine-methoxypolyethylene glycol (DSPE-MPEG).

In one or more embodiments, the sterol is one or more selected from the group consisting of cholesterol, 7-hydrocholesterol, lanosterol, sitosterol, brassicasterol, mycosterol, ostreasterol, stigmasterol, and ergosterol. In one or more preferred embodiments, the sterol is one or both selected from the group consisting of cholesterol and ergosterol. More preferably, in one or more embodiments, the sterol is cholesterol.

In one or more embodiments, the liposome composition comprises Utidelone and a zwitterionic phospholipid, such as the zwitterionic phospholipid as described above, preferably one or more selected from the group consisting of EPC, DOPC and DSPC. More preferably, in one or more embodiments, the liposome composition comprises Utidelone and DOPC.

In one or more embodiments, the liposome composition comprises Utidelone, a zwitterionic phospholipid, and a pegylated phospholipid. In one or more embodiments, the liposome composition comprises Utidelone, DSPE-MPEG, and a zwitterionic phospholipid, wherein the zwitterionic phospholipid is the zwitterionic phospholipid as described above, preferably one or more selected from the group consisting of HSPC, EPC, DOPC and DSPC, preferably one or more selected from the group consisting of HSPC, EPC and DOPC.

In one or more preferred embodiments, the liposome composition comprises Utidelone, a zwitterionic phospholipid and a sterol. More preferably, in one or more embodiments, the liposome composition comprises Utidelone, a zwitterionic phospholipid, and cholesterol, wherein the zwitterionic phospholipid is the zwitterionic phospholipid as described above, preferably one or more selected from the group consisting of HSPC, EPC, DOPC and DSPC, more preferably one or more selected from the group consisting of EPC, DOPC and DSPC. More preferably, in one or more embodiments, the liposome composition comprises Utidelone, EPC, and cholesterol. More preferably, in one or more embodiments, the liposome composition comprises Utidelone, DOPC, DSPC, and cholesterol.

In one or more preferred embodiments, the liposome composition comprises Utidelone, a pegylated phospholipid, a zwitterionic phospholipid, and a sterol. In one or more preferred embodiments, the liposome composition comprises Utidelone, DSPE-MPEG, a zwitterionic phospholipid, and cholesterol, wherein the zwitterionic phospholipid is the zwitterionic phospholipid as described above, preferably one or more selected from the group consisting of DOPC, DSPC, HSPC and EPC, and more preferably one or more selected from the group consisting of DOPC, HSPC and EPC. More preferably, in one or more embodiments, the liposome composition comprises Utidelone, DSPE-MPEG, DOPC, and cholesterol.

In one or more preferred embodiments, the liposome composition comprises Utidelone, an anionic phospholipid, a zwitterionic phospholipid, and a sterol. In one or more preferred embodiments, the liposome composition comprises Utidelone, DSPG, a zwitterionic phospholipid, and cholesterol, wherein the zwitterionic phospholipid is the zwitterionic phospholipid as described above, preferably one or more selected from the group consisting of DOPC, DSPC, HSPC and EPC, and more preferably one or more selected from the group consisting of DOPC, DSPC and EPC. More preferably, in one or more embodiments, the liposome composition comprises Utidelone, DSPG, DOPC, and cholesterol.

In an embodiment, the liposome composition does not include a liposome composition consisting of Utidelone and HSPC only.

In one or more embodiments, Utidelone is present in an amount of 2.5-20 wt%, preferably 3-10 wt%, based on the total weight of the liposome composition, in the liposome composition.

In one or more embodiments, the phospholipid is present in an amount of 40.0-97.5 wt%, preferably 65.0-97.0 wt%, based on the total weight of the liposome composition, in the liposome composition.

In one or more embodiments, the sterol, if present, is in an amount of 0.8-48.8 wt%, preferably 0.9-48.5 wt%, and more preferably 3-30 wt%, based on the total weight of the liposome composition, in the liposome composition.

In one or more embodiments, in the liposome composition wherein the phospholipid is a zwitterionic phospholipid in combination with a pegylated phospholipid, an anionic phospholipid and/or a cationic phospholipid, the zwitterionic phospholipid is present in an amount of 50-90 wt%; the pegylated phospholipid is present in an amount of 0-40 wt%; the anionic phospholipid is present in an amount of 0-40 wt%; and the cationic phospholipid is present in an amount of 0-40 wt%, based on the total weight of the phospholipid.

In one or more embodiments, in the liposome composition wherein the phospholipid is a zwitterionic phospholipid in combination with a pegylated phospholipid, the zwitterionic phospholipid is present in an amount of 60%-90%, preferably 70%-85%; and the pegylated phospholipid is present in an amount of 10-40 wt%, preferably 15%-30%, based on the total weight of the phospholipid.

In one or more embodiments, in the liposome composition wherein the phospholipid is a zwitterionic phospholipid in combination with an anionic phospholipid, the zwitterionic phospholipid is present in an amount of 60%-90%, preferably 65%-85%, and more preferably 70%-80%; and the anionic phospholipid is present in an amount of 10-40%, preferably 15%-35%, and more preferably 20%-30%, based on the total weight of the phospholipid.

In one or more embodiments, in the liposome composition wherein the phospholipid is a zwitterionic phospholipid in combination with a cationic phospholipid, the zwitterionic phospholipid is present in an amount of 60%-90%, preferably 65%-85%, and more preferably 70%-80%; and the cationic phospholipid is present in an amount of 10-40%, preferably 15%-35%, and more preferably 20%-30%, based on the total weight of the phospholipid.

In one or more embodiments, the mass ratio of Utidelone to the total phospholipid is in a range of from 0.2% to 30%, preferably from 2.6 to 25.3%, more preferably from 2.6% to 11.2%, and more preferably from 3.0 to 8.0%.

In one or more embodiments, in the liposome composition wherein the sterol is present, the mass ratio of the sterol to the total phospholipid is in a range of from 1% to 50%, preferably from 3% to 40%, and more preferably from 5% to 35%.

In one or more embodiments, the liposome composition further comprises one or more of an osmoregulator, an antioxidant, a preservative, a pH regulator, and a buffer. In one or more embodiments, the osmoregulator is one or more selected from the group consisting of sodium chloride, glycerin, sorbitol, mannitol, and glucose. In one or more embodiments, the pH regulator is one or more selected from the group consisting of sodium hydroxide, sodium citrate, citric acid, phosphoric acid, acetic acid, and hydrochloric acid. In one or more embodiments, the preservative is one or more selected from the group consisting of alkyl hydroxybenzoates, benzoic acid, sodium benzoate, sorbic acid, chlorhexidine acetate and benzalkonium bromide. In one or more embodiments, the antioxidant is one or more selected from the group consisting of sodium sulfite, sodium bisulfite, sodium metabisulfite, sodium thiosulfate, ascorbic acid, *tert*-butyl *p*-hydroxyanisole, 2,6-di-*tert*-butylated hydroxy toluene and vitamin E. In one or more embodiments, the buffer is one or more selected from the group consisting of citrate buffer, phosphate buffer, acetate buffer and Tris buffer.

In one or more embodiments, the liposome composition further comprises one of deionized water, 10% sucrose aqueous solution, phosphate buffer, and citrate buffer.

In one or more embodiments, the liposome composition is in a liquid form. Utidelone is present in a concentration of 0.01 mg/ml-20 mg/ml, and preferably 0.5 mg/ml-5 mg/ml, in the liposome composition in a liquid form.

In one or more embodiments, the liposome composition is in a form of lyophilized powder. In one or more embodiments, the liposome composition in the form of lyophilized powder may further comprise a lyoprotectant. In one or more embodiments, the lyoprotectant is one or more selected from the group consisting of glucose, sucrose, maltose, lactose, mannose, trehalose, glycine, and dextran.

In one or more embodiments, the liposomes in the liposome composition have a particle size of less than 250 nm, preferably of from 50 nm to 220 nm.

In one or more embodiments, the liposomes in the liposome composition have an average polydispersity index (PDI) of less than 0.3, preferably less than 0.2.

In one or more embodiments, the liposome composition of the present invention may be prepared by one or more methods selected from the group consisting of a shear mixing method, a thin-film rehydration method, a spray drying method, a freeze drying method, a freeze-thaw method, a solvent injection method, a reverse phase evaporation method, an emulsification-evaporation method, a microfluidic method, an ultrasonication method, a supercritical fluid method, and a homogenization method.

In one or more embodiments, the liposome composition of the present invention may be prepared by a thin-film rehydration method. In one or more embodiments, the thin-film rehydration method comprises the steps of:

(1) mixing Utidelone, a phospholipid, and optionally a sterol with a solvent uniformly to obtain a mixed solution;
(2) evaporating the mixed solution obtained in step (1) under reduced pressure to obtain a Utidelone-containing lipid film;
(3) hydrating the lipid film obtained in step (2) to obtain a liposome solution; and
(4) subjecting the liposome solution obtained in step (3) to granule sizing to obtain a nanoliposome solution.

In one or more embodiments, the solvent used in step (1) is an organic solvent or a mixture of an organic solvent and water. In one or more embodiments, the organic solvent is one or more selected from the group consisting of chloroform, dichloromethane, *tert*-butanol, isopropanol, ethyl acetate, ethanol, methanol, tetrahydrofuran, dioxane, acetonitrile, acetone, dimethyl sulfoxide, dimethylformamide, and methylpyrrolidone.

In one or more embodiments, the solvent used in step (1) is selected from the group consisting of a mixture of dichloromethane and methanol; a mixture of dichloromethane and ethanol; and a mixture of chloroform, methanol and water.

In one or more embodiments, the solvent used in step (1) is selected from the group consisting of a mixture of methylene chloride and methanol in a ratio of 5:1 or 6:1; a mixture of methylene chloride and ethanol in a ratio of 5:1; and a mixture of chloroform, methanol and water in a ratio of 95:4:1.

In one or more embodiments, a hydrating solvent is used to hydrate the lipid film in step (3). In one or more embodiments, the hydrating solvent is one or more of deionized water, 10% sucrose aqueous solution, a phosphate buffer, and a citrate buffer. In one or more embodiments, the hydrating solvent further comprises one or more of an osmoregulator, an antioxidant, a preservative, a pH regulator, and a buffer.

In one or more embodiments, the granule sizing in step (4) is carried out by using one or more methods selected from the group consisting of a shearing method, a high-pressure homogenization method and a microfluidization homogenization method.

In one or more embodiments, the thin-film rehydration method further comprises step (5) of sterilizing the nanoliposome solution obtained in step (4).

In one or more embodiments, the thin-film rehydration method further comprises a step of lyophilizing the drug-containing solution obtained in step (5).

In another aspect, the present invention provides use of the liposome composition according to the present invention in the manufacture of a medicament for preventing or treating a cancer.

In one or more embodiments, the cancer is a solid tumor, such as a breast cancer, a lung cancer, and a gastrointestinal tumor.

In one or more embodiments, the liposome composition is prepared into a pharmaceutical formulation. In one or more embodiments, the liposome composition is prepared into a pharmaceutical formulation for parenteral, inhalation, intraperitoneal, intravesical, intramuscular, intravenous, intratracheal, subcutaneous, intraocular, intrathecal, transdermal, rectal or intravaginal administration. In one or more preferred embodiments, the liposome composition is prepared into a pharmaceutical formulation for intravenous administration. In one or more preferred embodiments, the pharmaceutical formulation is a solid preparation, a liquid preparation or a gas preparation, more preferably a liquid preparation for injection. In one or more preferred embodiments, the pharmaceutical formulation is a stable aqueous suspension reconstituted from a sterile lyophilized powder of the liposome composition before use.

In one or more embodiments, the pharmaceutical formulation prepared from the liposome composition also comprises an additional drug. In one or more embodiments, the additional drug is an anticancer drug.

The technical solutions of the present invention have the following beneficial effects:

The invention provides a stable Utidelone liposome composition.

The Utidelone liposome composition of the present invention does not comprise excipients that are likely to cause allergic reactions in human body, and can effectively eliminate the allergic reactions caused by the castor oil and the irritant reactions caused by organic solvents in the existing clinical preparations, thereby improving the patients' drug compliance, while prolonging the *in vivo* half-life of Utidelone, so as to achieve the clinical effects of attenuating toxicity and increasing efficacy.

The formula combinations adopted by the present invention can increase the weight ratio of Utidelone in the liposomes to over 3%, which means an increase in drug loading capacity, and the drug loading capacity of the liposomes in the present application can reach over 3% or even up to 7%, which is much higher than the drug loading capacity of the existing Utidelone preparations. Increase of the drug loading capacity can reduce the amount of excipients, lower production costs, reduce the burden of medication for patients, while reducing the potential side effects caused by excipients.

The Utidelone liposome composition of the present invention has a high drug loading capacity and stability, thereby having a good prospect for industrialization.

## Description of Drawings

Fig. 1 shows a diagram of the hydrated particle size distribution of the Utidelone liposomes prepared in Example 1.
Fig. 2 shows the results of the *in vitro* release rate of the Utidelone liposomes prepared in Example 5.
Fig. 3 shows a diagram of the hydrated particle size distribution of the Utidelone liposomes prepared in Example 6.
Fig. 4 shows a diagram of the hydrated particle size distribution of the Utidelone liposomes prepared in Example 10.
Fig. 5 shows a graph for characterizing the morphology of the Utidelone liposomes prepared in Example 17.

## Embodiments

The following examples are intended to further illustrate the present invention, but not to limit the scope of the present invention. The present invention is further described in detail below in conjunction with the examples. It will be appreciated by those skilled in the art that the present invention is not limited to these examples and the preparation methods used therein. Moreover, equivalent substitutions, combinations, improvements or modifications to the present invention may be carried out by those skilled in the art from the disclosure of the present invention, and all of these will be included in the scope of the present invention.

## Test Example 1: Determination of particle size of the liposomes

The particle size of liposomes was determined by dynamic light scattering (DLS) using Malvern Zetasizer NANO ZS90. Detection mode: automatic; Refractive index of the sample to be tested: 1.340; Dispersion medium: ultrapure water; Temperature 25°C; Viscosity: 0.8872cp; Refractive index of dispersion medium: 1.330. These conditions were used to get a particle size diagram.

## Test Example 2: Assay of Utidelone in the liposomes

The assay was carried out according to the high performance liquid chromatography (Chinese Pharmacopoeia 2020 Part IV General Rules 0512).

Column: GL Sciences Intertsil ODS-3 5 $\mu$m, 4.6*250 mm
Column temperature: 30°C;
Running time: 25 min;
Flow rate: 1.0 ml/min;
Injection volume: 20 $\mu$l;
Sample tray temperature: 25°C;
Mobile phase: water-acetonitrile (40:60);
Detection wavelength: 250 nm.
Reference solutions: the Utilidelon reference stock solution was diluted with acetonitrile to a series of concentrations: 10 $\mu$g/ml, 20 $\mu$g/ml, 50 $\mu$g/ml, 100 $\mu$g/ml, and 200 $\mu$g/ml.
Test solutions: the test solutions were obtained by taking each of the samples to be tested in a sample flask, adding thereto an appropriate amount of 75% isopropanol, shaking the flask to dissolve the sample, transferring all the solution into a 10-ml volumetric flask, then using 75% isopropanol to wash the sample flask, transferring the washed solution to the volumetric flask, adjusting the volume to constant volume, shaking the solution well, and filtering the solution with a 0.22-$\mu$m organic filter.

20 $\mu$l of each of the solutions was measured accurately, and injected into the liquid chromatograph to get the chromatogram. The contents of Utidelone in the test solutions were calculated according to the linear equation.

**Test Example 3: Determination of stability of Utidelone liposomes**

The filtered liposome solution to be tested was divided into vials, which were placed in a lyophilizer (Virtis AdVantage) for lyophilization. The parameters for lyophilization were: pre-freezing at -45°C for 120 minutes, first drying at -30°C for 1000 minutes, and second drying at 25°C for 720 minutes. The freeze-dried product was stored in a refrigerator at 2-8°C. At the set time, the freeze-dried products were taken out, redissolved in 75% isopropanol, and tested for their particle size and contents.

**Test Example 4 Determination of encapsulation efficiency of Utidelone liposomes**

Preparation of the test solution (for the amount of encapsulated drug): 0.2 ml of the Utidelone liposome solution to be tested was loaded on a Sephadex G-50 column for separation, and eluted by first using 30ml of PBS (6.8) as an eluent. The first 15ml of eluate was collected, and determined for the content of Utidelone therein as the amount of encapsulated drug. Then the remaining free drug was eluted by using 30 ml of physiological sodium chloride solution.

Preparation of the test solution (for the total drug amount): Another 0.2 ml of Utidelone liposome solution to be tested was dissolved and diluted to 10ml with 60% acetonitrile, shaken well, filtered, and determined for the content of Utidelone therein which was the total amount of the encapsulated and unencapsulated drug.

The encapsulation efficiency was calculated following the formula:

Encapsulation efficiency (%) = amount of the drug encapsulated in --> liposomes /total drug amount $\times$ 100.

**Test Example 5 Determination of the *in vitro* release rate of Utidelone liposomes**

The method for determining the release rate is as follows:

1. Instruments, reagents and samples

A fully automatic dissolution instrument, an electronic analytical balance, a high performance liquid chromatograph, a pH meter, Utidelone reference substance, acetonitrile (chromatographically pure), and phosphate buffer (pH 7.4).

2. Chromatographic conditions:

Column: Agilent ZORBAX Eclipse plus C18, 4.6 mm$\times$150 mm, 3 um
Mobile Phase A: Water
Mobile Phase B: Acetonitrile
Detection wavelength: 250 nm
Flow rate: 1.0 ml/min
Column temperature: 30°C
Injection volume: 50 ul
Mobile phase: acetonitrile-water (60:40)

3. Preparation of solutions

Blank solution: phosphate buffer (pH7.4): acetonitrile = 1:1
Reference solution: an appropriate amount of Utidelone reference substance was weighed accurately, put into a volumetric flask, dissolved with the blank solution and diluted to 0.05 $\mu$g/ml.
Preparation of test solution: 0.5 ml of the Utidelone liposome solution to be tested was pipetted precisely, and placed in a dialysis bag. Then the two sections were tied tightly. 500 ml of phosphate buffered saline (pH7.4) was used as the release medium, with a rotation speed of 100 rpm and at a temperature of $37 \pm 0.5$ °C, the release medium was taken at 0.5 h, 1.0 h, 2.0 h, 3.0 h, 4.0 h, 6.0 h, 10.0 h, 14.0 h, 18.0 h, 20.0 h, 22.0 h, and 24.0 h. The initial filtrate is 4 ml, the sampling volume is 8 ml, and at the same time, 12.0 mL of blank release medium was added. 1 ml of the sample at each sampling point was pipetted accurately in a beaker, and then added with 1ml of acetonitrile to mix well, filtered, processed and then injected for analysis. After the system was equilibrated, samples were injected sequentially to get the chromatograms, and the test results were recorded in the relevant notes. The cumulative release was calculated following the formula.

$$\text{Cumulative release} = \frac{A_s \times C_r \times V}{A_r \times M} \times 100\% + (R_0 + R_1 + \cdots R_n) \times \frac{V1}{V2}$$

wherein:

$A_s$: the peak area of Utidelone in the test solution;
$C_r$: the concentration in μg/ml in the reference solution;
$V$: the dilution factor of the test solution, 1000;
$A_r$: the average peak area of Utidelone in the reference solution;
M: the total amount of Utidelone put into the release medium;
$V_1$: the sampling volume for the release test, 12 mL;
$V_2$: the volume of the dissolution medium, 500 mL.

**Test Example 6: Determination of Drug Loading Capacity for Utidelone Liposomes**

The assay of Utidelone (w/v%) in the Utidelone liposomes was carried out according to the method as described in Test Example 2.

The assays of the phospholipid and the sterol in the Utidelone liposomes were carried out by the following method:

Detecting Instrument: Waters Arc HPLC (2424 ELSD)

Chromatographic conditions:

Mobile phase A: methanol-tetrahydrofuran-0.17 mol/L ammonium acetate aqueous solution (89:10:1), Mobile phase B: 4 mM ammonium acetate aqueous solution. Isocratic elution was carried out in a ratio of mobile phase A to mobile phase B of 98.5:1.5. Flow rate: 1.0 ml/min; Column: Narochrom chromcore 120 C18 (3 μm, 4.6 × 150 mm); Column temperature 30 °C; Drift tube temperature: 45 °C, Atomization temperature: 36 °C, Gas flow rate: 40 psi, and gain: 100.

Reference solutions: An appropriate amount of the phospholipid or sterol reference substance was weighed accurately, dissolved and diluted with methanol to form a series of linear reference solutions.

Test solutions: 0.5ml of the Utidelone liposome solution to be tested was placed into a 10-ml volumetric flask, dissolved by ultrasonic emulsion breaking with methanol, adjusted the volume to the constant volume, and filtered through a 0.45-μm microporous membrane for use.

10 μl of each of the solutions was taken accurately, and injected into the liquid chromatograph to get the chromatograms. The amounts (w/v %) of the phospholipid and the sterol in the test solutions were calculated according to the equation.

The drug loading capacity of the Utidelone liposomes was calculated following the formula:

Drug loading capacity of Utidelone liposomes = amount of Utidelone /(amount of Utidelone + total amount of phospholipid + amount of sterol)

**Comparative Example 1**

5.3 mg of Utidelone and 152mg of HSPC were weighted, dissolved with 1.5 ml of organic solvents (dichloromethane and methanol in a ratio of 5:1), and formed a lipid film by removing the organic solvents using rotatory evaporation (IKA, RV10) at 65°C. The lipid film was hydrated with 20ml of a hydrating solvent (phosphate buffer (pH 6.54), 70°C). After the hydration, the hydrated solution was ultrasonically homogenized using a probe ultrasonic instrument (Scientz). After the homogenization, the drug-containing solution was turbid. Obvious precipitation was visible to the naked eyes. The liposome solution was filtered using a 0.22-μm polyethersulfone filter membrane. The hydrated particle size of the liposomes was determined by dynamic light scattering (DLS) to be 147.3 nm with a polydispersity index (PDI) of 0.203. The encapsulation efficiency was greater than 90%.

## Example 1

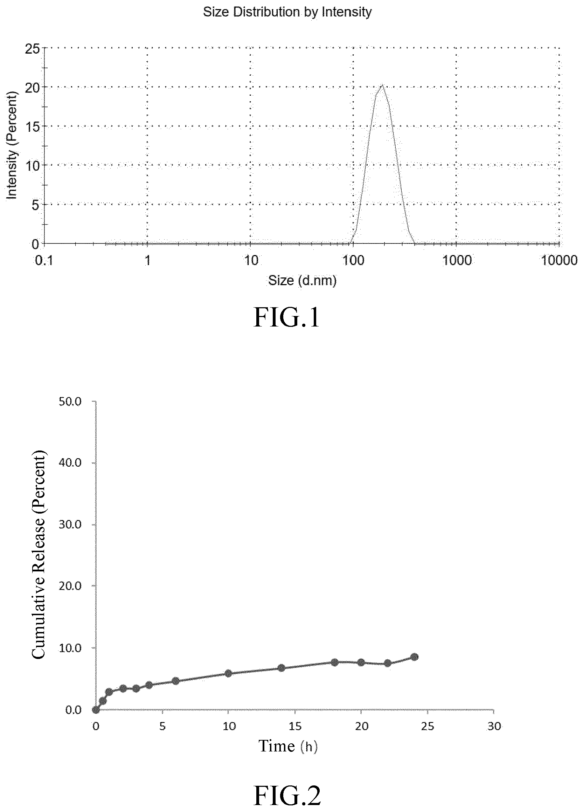

12.5 mg of Utidelone, 201 mg of DOPC, 68 mg of DSPE-MPEG, and 34 mg of cholesterol were weighted, charged into a flask, then dissolved by addition of 4 ml of organic solvents (dichloromethane and methanol in a ratio of 5:1), and formed a lipid film by removing the organic solvents using rotatory evaporation (IKA, RV10) at 40 °C. The lipid film was hydrated with a hydrating solvent (citrate buffer (pH 5.54) containing 10% sucrose, 6 ml) at 55-60 °C. After the hydration, the hydrated solution was extruded 10-15 times by using an extruder (Avanti) with a 100-nm extrusion film. After the extrusion was completed, the liposome solution was filtered using a 0.22-$\mu$m polyethersulfone filter membrane. The hydrated particle size of the liposomes was determined by dynamic light scattering (DLS). The particle size diagram is shown in Figure 1, with an average particle size of 181.3 nm, and a PDI of 0.067.

## Example 2

6.2 mg of Utidelone, 82.5 mg of DOPC, 30mg of DSPG, and 15 mg of cholesterol were weighted, charged into a flask, then dissolved by addition of 2 ml of organic solvents (chloroform, methanol and water in a ratio of 95:4:1), and formed a lipid film by removing the organic solvents using rotatory evaporation (IKA, RV10) at 40°C. The lipid film was hydrated with a hydrating solvent (10% sucrose solution, 5 ml) at 40-45°C. After the hydration, the hydrated solution was extruded 10-15 times by using an extruder (Avanti) with a 100-nm extrusion film. After the extrusion was completed, the liposome solution was filtered using a 0.22-$\mu$m polyethersulfone filter membrane. The filtered drug-containing solution was divided into vials, which were put into a lyophilizer for lyophilization, and the lyophilization procedure was as described in Test Example 3. The freeze-dried product was stored in a refrigerator at 2-8°C, and at the set time, the freeze-dried product was taken out and redissolved to determine for the particle size and the contents. The results are shown in Table 1.

Table 1

|  | Average particle size (nm) | Polydispersity index | Content (%) |
|---|---|---|---|
| 0 day | 181.00 | 0.098 | 100.00 |
| 7 days | 175.5 | 0.059 | 95.49 |
| 14 days | 167 | 0.057 | 103.04 |
| 1 month | 181.6 | 0.101 | 101.36 |
| 2 months | 177.3 | 0.082 | 98.48 |

As can be seen from the data in Table 1, the Utidelone liposomes prepared according to the present invention have an extremely high stability, and the drug content is still close to 100 % when stored at 2-8°C for up to two months. There are no significant changes in particle size and in particle size distribution.

## Example 3

9.1 mg of Utidelone, 101 mg of DSPC, 30 mg of DSPG, and 40 mg of cholesterol were weighted, charged into a flask, then dissolved by addition of 3 ml of organic solvents (chloroform, methanol and water in a ratio of 95:4:1), and formed a lipid film by removing the organic solvents using rotatory evaporation (IKA, RV10) at 70°C. The lipid film was hydrated with 20 ml of a hydrating solvent (phosphate buffer (pH 6.54), 70°C). After the hydration, the hydrated solution was homogenized 15-25 times using a homogenizer (ATS) with a homogenization pressure of 1200-1500 bar and a homogenization temperature of 60-70°C. After the homogenization was completed, the liposome solution was filtered using a 0.22-$\mu$m polyethersulfone filter membrane. The hydrated particle size of the liposomes was determined by using dynamic light scattering (DLS) to be 57.97 nm, with a polydispersity index (PDI) of 0.078. The amount of Utidelone was 0.01 mg/ml, and the encapsulation efficiency was greater than 90%.

## Example 4

5.3 mg of Utidelone, 122 mg of HSPC, and 32 mg of DSPE-MPEG were weighted, charged into a flask, then dissolved by addition of 2 ml of organic solvents (dichloromethane and methanol in a ratio of 5:1), and formed a lipid film by removing the organic solvents using rotatory evaporation (IKA, RV10) at 70°C. The lipid film was hydrated with 20 ml of a hydrating solvent (phosphate buffer (pH 6.54), 70°C). After the hydration, the hydrated solution was homogenized 15-25 times using a homogenizer (ATS) with a homogenization pressure of 1200-1500bar and a homogenization temperature of 60-70°C. After the homogenization was completed, the liposome solution was filtered using a 0.22-$\mu$m polyethersulfone filter membrane. The hydrated particle size of the liposomes was determined by using dynamic light scattering (DLS) to be 61.33 nm with a polydispersity index (PDI) of 0.168. The amount of Utidelone was 0.127 mg/ml, and the encapsulation efficiency was greater than 90%.

**Example 5**

5.0 mg of Utidelone, 119 mg of HSPC, and 32 mg of DSPE-MPEG were weighted, dissolved with 1.5 ml of organic solvents (dichloromethane and methanol in a ratio of 5:1), and formed a lipid film by removing the organic solvents using rotatory evaporation (IKA, RV10) at 65°C. The lipid film was hydrated with a hydrating solvent (phosphate buffer (pH 6.54), 20 ml) at 70°C. After the hydration, the hydrated solution was ultrasonically homogenized using a probe ultrasonic instrument (Scientz). After the homogenization was completed, the liposome solution was filtered using a 0.22-$\mu$m polyethersulfone filter membrane. The hydrated particle size of the liposomes was determined by using dynamic light scattering (DLS) to be 99.13 nm, with a polydispersity index (PDI) of 0.221. The obtained liposomes were subjected to the determination of the in vitro release rate of Utidelone liposomes by using the procedure described in Test Example 5, and the results are shown in Fig. 2. It has been preliminarily shown by the *in vitro* test that the Utidelone liposome of the present invention has a sustained-release effect.

**Example 6**

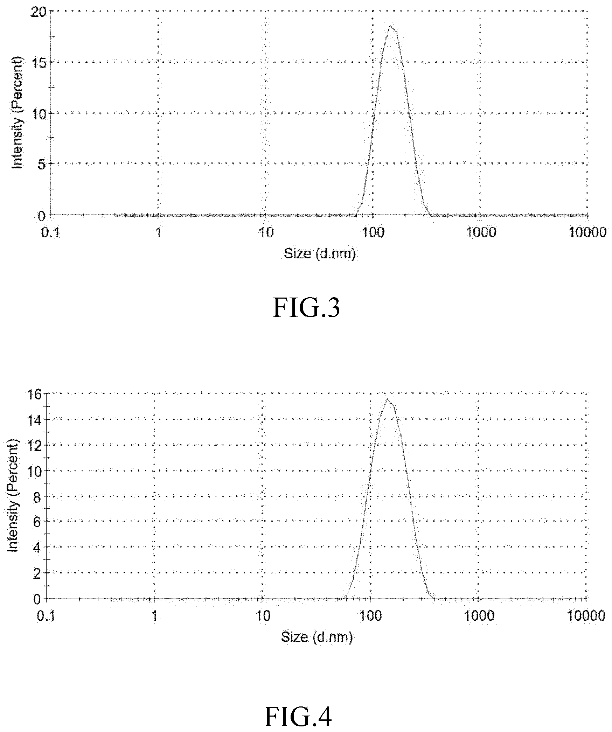

5.6 mg of Utidelone, 103 mg of EPC, and 30 mg of cholesterol were weighted, charged into a flask, then dissolved by addition of 2 ml of organic solvents (dichloromethane and methanol in a ratio of 5:1), and formed a lipid film by removing the organic solvents using rotatory evaporation (IKA, RV10) at 40°C. The lipid film was hydrated with a hydrating solvent (10% sucrose solution, 3 ml) at 50-55 °C. After the hydration, the hydrated solution was extruded 10-20 times using an extruder (Avanti) with a 100-nm extrusion film. After the extrusion was completed, the liposome solution was filtered using a 0.22-$\mu$m polyethersulfone filter membrane. The hydrated particle size of the liposomes was determined by using dynamic light scattering (DLS) to be 144.5 nm, with a polydispersity index (PDI) of 0.069. The particle size diagram is shown in Figure 3. The encapsulation efficiency was 94.3%.

**Example 7**

5 mg of Utidelone and 105 mg of DOPC were weighted, charged into a flask, then dissolved by addition of 2 ml of organic solvents (dichloromethane and methanol in a ratio of 5:1), and formed a lipid film by removing the organic solvents using rotatory evaporation (IKA, RV10) at 40°C. The lipid film was hydrated with a hydrating solvent (10% sucrose solution, 3 ml) at 50-55°C. After the hydration, the hydrated solution was extruded 10-20 times using an extruder (Avanti) with a 100-nm extrusion film. After the extrusion was completed, the liposome solution was filtered using a 0.22-$\mu$m polyethersulfone filter membrane. The hydrated particle size of the liposomes was determined by using dynamic light scattering (DLS) to be 193.2 nm, with a polydispersity index (PDI) of 0.143. The amount of Utidelone was 1.624 mg/ml, and the encapsulation efficiency was greater than 90%.

**Example 8**

5.6 mg of Utidelone, 20 mg of DSPE-MPEG and 104 mg of DOPC were weighted, charged into a flask, then dissolved by addition of 2 ml of organic solvents (dichloromethane and methanol in a ratio of 5:1), and formed a lipid film by removing the organic solvents using rotatory evaporation (IKA, RV10) at 40°C. The lipid film was hydrated with a hydrating solvent (phosphate buffer (pH 6.54), 3 ml) at 50-55°C. After the hydration, the hydrated solution was extruded 10-20 times using an extruder (Avanti) with a 100-nm extrusion film. After the extrusion was completed, the liposome solution was filtered using a 0.22-$\mu$m polyethersulfone filter membrane. The hydrated particle size of the liposomes was determined by using dynamic light scattering (DLS) to be 196.9 nm, with a polydispersity index (PDI) of 0.066. The amount of Utidelone was 1.717 mg/ml, and the encapsulation efficiency was greater than 90%.

**Example 9**

5 mg of Utidelone, 22 mg of DSPE-MPEG and 104 mg of EPC were weighted, charged into a flask, then dissolved by addition of 2 ml of organic solvents (dichloromethane and methanol in a ratio of 5:1), and formed a lipid film by removing the organic solvents using rotatory evaporation (IKA, RV10) at 40°C. The lipid film was hydrated with a hydrating solvent (phosphate buffer (pH 6.54), 3 ml) at 50-55°C. After the hydration, the hydrated solution was extruded 10-20 times using an extruder (Avanti) with a 100-nm extrusion film. After the extrusion was completed, the liposome solution was filtered using a 0.22-$\mu$m polyethersulfone filter membrane. The hydrated particle size of the liposomes was determined by using dynamic light scattering (DLS) to be 155.8 nm, with a polydispersity index (PDI) of 0.079. The encapsulation efficiency was greater than 90%.

**Example 10**

3.12 mg of Utidelone, 53 mg of DOPC, 17 mg of DSPE-MPEG and 7 mg of cholesterol were weighted, charged into a flask, then dissolved by addition of 0.6 ml of organic solvents (dichloromethane and ethanol in a ratio of 5:1), and formed a lipid film by removing the organic solvents using rotatory evaporation (IKA, RV10) at 40°C. The lipid film was hydrated with a hydrating solvent (10% sucrose solution, 3 ml) at 40-50°C. After the hydration, the hydrated solution was extruded 10-15 times using an extruder (Avanti) with a 100-nm extrusion film. After the extrusion was completed, the liposome solution was filtered using a 0.22-$\mu$m polyethersulfone filter membrane. The hydrated particle size of the liposomes was determined by using dynamic light scattering (DLS) to be 136.9 nm, with a polydispersity index (PDI) of 0.101. The particle size diagram is shown in Figure 4. The encapsulation efficiency was greater than 90%.

**Example 11**

6.5 mg of Utidelone, 100 mg of DOPC, 35 mg of DSPC and 17 mg of cholesterol were weighted, charged into a flask, then dissolved by addition of 2 ml of organic solvents (dichloromethane and methanol in a ratio of 5:1), and formed a lipid film by removing the organic solvents using rotatory evaporation (IKA, RV10) at 40°C. The lipid film was hydrated with a hydrating solvent (citrate buffer (pH 5.54), 3 ml) at 50-55°C. After the hydration, the hydrated solution was extruded 10-15 times using an extruder (Avanti) with a 100-nm extrusion film. After the extrusion was completed, the liposome solution was filtered using a 0.22-$\mu$m polyethersulfone filter membrane. The hydrated particle size of the liposomes was determined by using dynamic light scattering (DLS) to be 201.9 nm, with a polydispersity index (PDI) of 0.188. The amount of Utidelone was 2.061 mg/ml, and the encapsulation efficiency was greater than 90%.

**Example 12**

3.3 mg of Utidelone, 52 mg of DOPC, 17 mg of DSPG and 5 mg of cholesterol were weighted, charged into a flask, then dissolved by addition of 2 ml of organic solvents (chloroform, methanol and water in a ratio of 95:4:1), and formed a lipid film by removing the organic solvents using rotatory evaporation (IKA, RV10) at 40°C. The lipid film was hydrated with a hydrating solvent (10% sucrose solution, 3 ml) at 35-40 °C. After the hydration, the hydrated solution was extruded 10-15 times using an extruder (Avanti) with a 100-nm extrusion film. After the extrusion was completed, the liposome solution was filtered using a 0.22-$\mu$m polyethersulfone filter membrane. The hydrated particle size of the liposomes was determined by using dynamic light scattering (DLS) to be 144.4 nm, with a polydispersity index (PDI) of 0.174. The encapsulation efficiency was greater than 90%.

**Example 13**

3.5 mg of Utidelone, 53 mg of EPC, 17 mg of DSPG and 4 mg of cholesterol were weighted, charged into a flask, then dissolved by addition of 2 ml of organic solvents (chloroform, methanol and water in a ratio of 95:4:1), and formed a lipid film by removing the organic solvents using rotatory evaporation (IKA, RV10) at 40°C. The lipid film was hydrated with a hydrating solvent (phosphate buffer (pH 6.54), 3 ml) at 25-35°C. After the hydration, the hydrated solution was extruded 10-15 times using an extruder (Avanti) with a 100-nm extrusion film. After the extrusion was completed, the liposome solution was filtered using a 0.22-$\mu$m polyethersulfone filter membrane. The hydrated particle size of the liposomes was determined by using dynamic light scattering (DLS) to be 147.3 nm, with a polydispersity index (PDI) of 0.155. The encapsulation efficiency was greater than 90%.

**Example 14**

3 mg of Utidelone, 50 mg of EPC, 17 mg of DSPE-PEG and 4.5 mg of cholesterol were weighted, charged into a flask, then dissolved by addition of 2 ml of organic solvents (chloroform, methanol and water in a ratio of 95:4:1), and formed a lipid film by removing the organic solvents using rotatory evaporation (IKA, RV10) at 40°C. The lipid film was hydrated with a hydrating solvent (10% sucrose solution, 3 ml) at 25-35°C. After the hydration, the hydrated solution was extruded 10-15 times using an extruder (Avanti) with a 100-nm extrusion film. After the extrusion was completed, the liposome solution was filtered using a 0.22-$\mu$m polyethersulfone filter membrane. The hydrated particle size of the liposomes was determined by using dynamic light scattering (DLS) to be 134.3 nm, with a polydispersity index (PDI) of 0.11. The encapsulation efficiency was greater than 90%.

**Example 15**

5.2 mg of Utidelone, 85.2 mg of EPC, 27.5 mg of DSPG and 7.7 mg of cholesterol were weighted, charged into a flask, then dissolved by addition of 3 ml of organic solvents (chloroform, methanol and water in a ratio of 95:4:1), and formed a lipid film by removing the organic solvents using rotatory evaporation (IKA, RV10) at 40°C. The lipid film was hydrated with a hydrating solvent (10% sucrose solution, 5 ml) at 40-50°C. After the hydration, the hydrated solution was extruded 10-15 times using an extruder (Avanti) with a 100-nm extrusion film. After the extrusion was completed, the liposome solution was filtered using a 0.22-μm polyethersulfone filter membrane. The filtered drug-containing solution was divided into vials, which were put into a lyophilizer for lyophilization. The parameters for lyophilization were: pre-freezing at -45°C for 120 minutes, first drying at -30°C for 1000 minutes, and second drying at 25°C for 720 minutes. After the freeze-dried product was redissolved, the hydrated particle size of the liposomes was determined by using dynamic light scattering (DLS) to be 183.4 nm, with a polydispersity index (PDI) of 0.163. The amount of Utidelone was 0.797 mg/ml, and the encapsulation efficiency was greater than 90%.

**Example 16**

5 mg of Utidelone, 86 mg of EPC, 28 mg of DSPE-PEG and 8 mg of cholesterol were weighted, charged into a flask, then dissolved by addition of 2 ml of organic solvents (dichloromethane and methanol in a ratio of 5:1), and formed a lipid film by removing the organic solvents using rotatory evaporation (IKA, RV10) at 40°C. The lipid film was hydrated with a hydrating solvent (10% sucrose solution, 5 ml) at 40-50°C. After the hydration, the hydrated solution was extruded 10-15 times using an extruder (Avanti) with a 100-nm extrusion film. After the extrusion was completed, the liposome solution was filtered using a 0.22-μm polyethersulfone filter membrane. The filtered drug-containing solution was divided into vials, which were put into a lyophilizer for lyophilization. The parameters for lyophilization were: pre-freezing at -45°C for 120 minutes, first drying at -30°C for 1000 minutes, and second drying at 25°C for 720 minutes. After the freeze-dried product was redissolved, the hydrated particle size of the liposomes was determined by using dynamic light scattering (DLS) to be 173.1 nm, with a polydispersity index (PDI) of 0.129. The amount of Utidelone was 0.799 mg/ml, and the encapsulation efficiency was greater than 90%.

**Example 17**

6.7 mg of Utidelone, 115.9 mg of DOPC, 33.2 mg of DSPE-MPEG and 16.7 mg of cholesterol were weighted, charged into a flask, then dissolved by addition of 2.4 ml of organic solvents (dichloromethane and ethanol in a ratio of 5:1), further added with 3 g of glass beads (ASONE, 0.350-0.500 mm, BZ04), and formed a lipid film by removing the organic solvents using rotatory evaporation (Buchi, R300) at 40°C. The lipid film was hydrated with a hydrating solvent (purified water, 6 ml) at 25-35°C. After the hydration, the hydrated solution was extruded 10-15 times using an extruder (ATS, AE001) with a 50-nm polycarbonate film (Whatman). After the extrusion was completed, the liposome solution was filtered using a 0.22-μm polyethersulfone filter membrane to obtain a liposome solution. The hydrated particle size of the liposomes was determined by using dynamic light scattering (DLS) to be 82.3 nm, with a polydispersity index (PDI) of 0.075.

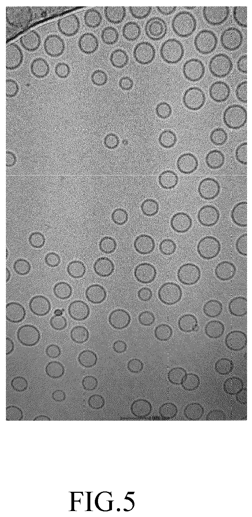

The morphology of the obtained Utidelone liposomes was characterized using a cryo-transmission electron microscope (Talos-F200C). As shown in Fig. 5, the liposomes have a saclike structure with a particle size of less than 100 nm.

The obtained Utidelone liposome solution was determined by using the method as described in Test Example 6, to have an amount of Utidelone of 0.84 mg/ml, a total amount of DOPC and DSPE-MPEG of 14.55 mg/ml, and an amount of cholesterol of 1.67 mg/ml. Calculated following the formula as described, the drug loading capacity of Utidelone liposome was 4.9% (w/w %).

As can be seen from the above Examples, the Utidelone liposome compositions of the present invention have a small particle size with an even and normal distribution; have an encapsulation efficiency of greater than 90%; and have an extremely high stability. The drug content is still close to 100% when stored at 2-8°C for up to two months, and there are no significant changes in particle size and in particle size distribution. The Utidelone liposome compositions of the present invention have a sustained-release effect and prolong the action time; have a high drug loading capacity, which can reach over 3% or even up to 7%, far exceeding the drug loading capacity of the existing Utidelone formulations, thereby being able to reduce the amounts of excipients, reducing the toxicity associated with excipients, and making it easier to meet clinical needs.

**Claims**

1.  A liposome composition comprising Utidelone, a phospholipid, and optionally, a sterol.

2.  The liposome composition according to claim 1, wherein the phospholipid is one or more selected from the group consisting of a pegylated phospholipid, an anionic phospholipid, a cationic phospholipid, and a zwitterionic phospholipid, or wherein the phospholipid is one or more selected from the group consisting of a pegylated phospholipid, an anionic phospholipid, and a zwitterionic phospholipid; preferably, the phospholipid is selected from the group consisting of a combination of a pegylated phospholipid and a zwitterionic phospholipid, and a combination of an anionic phospholipid and a zwitterionic phospholipid.

3.  The liposome composition according to claim 2, wherein the pegylated phospholipid is one or more selected from the group consisting of distearoyl phosphatidylethanolamine-polyethylene glycol (DSPE-PEG), distearoyl phosphatidylethanolamine-methoxypolyethylene glycol (DSPE-MPEG), dimyristoyl phosphatidylethanolamine-polyethylene glycol (DMPE-PEG), dipalmitoylglycero succinate polyethylene glycol (DPGS-PEG), cholesteryl-pegylated phospholipid, and ceramido pegylated phospholipid; preferably selected from the group consisting of distearoyl phosphatidylethanolamine-polyethylene glycol (DSPE-PEG), and distearoyl phosphatidylethanolamine-methoxypolyethylene glycol (DSPE-MPEG); and more preferably distearoyl phosphatidylethanolamine-methoxypolyethylene glycol (DSPE-MPEG); or

    wherein the anionic phospholipid is one or more selected from the group consisting of phosphatidylglycerol, di(hexadecyl) phosphate (DhP), phosphatidylinositol, phosphatidylserine, phosphatidylglycerol, lysophosphatidylglycerol (lysylphosphatidylglycerol, LPG), phosphatidylethanolamine, phosphatidic acid, cardiolipin, and cholesteryl hemisuccinate; preferably phosphatidylglycerol; preferably, the phosphatidylglycerol is one or more selected from the group consisting of dimyristoyl phosphatidylglycerol (DMPG), dipalmitoyl phosphatidylglycerol (DPPG), distearoyl phosphatidylglycerol (DSPG), dioleoyl phosphatidylglycerol (DOPG), dilauroyl phosphatidylglycerol (DLPG), egg phosphatidylglycerol (EPG), egg phosphatidylinositol (EPI), soybean phosphatidylglycerol (SPG), soybean phosphatidylinositol (SPI), hydrogenated egg phosphatidylglycerol (HEPG), hydrogenated soybean phosphatidylglycerol (HSPG), hydrogenated egg phosphatidylinositol (HEPI), palmitoyl stearoyl phosphatidylglycerol (PSPG), and hydrogenated soybean phosphatidylinositol (HSPI); preferably one or more selected from the group consisting of distearoyl phosphatidylglycerol (DSPG), hydrogenated egg phosphatidylglycerol (HEPG), and hydrogenated soybean phosphatidylglycerol (HSPG); and preferably distearoyl phosphatidylglycerol (DSPG); or
    wherein the zwitterionic phospholipid is one or more selected from the group consisting of egg phosphatidylcholine (EPC), egg phosphatidylserine (EPS), phosphatidylethanolamine (EPE), soybean phosphatidylserine (SPS), soybean phosphatidylethanolamine (SPE), hydrogenated egg phosphatidylcholine (HEPC), hydrogenated egg phosphatidylserine (HEPS), hydrogenated egg phosphatidylethanolamine (HEPE), hydrogenated soybean phosphatidylcholine (HSPC), hydrogenated soybean phosphatidylserine (HSPS), hydrogenated soybean phosphatidylethanolamine (HSPE), dipalmitoyl phosphatidylcholine (DPPC), 1-palmitoyl-2-myristoylphosphatidylcholine (PMPC), 1-myristoyl-2-palmitoylphosphatidylcholine (MPPC), dioleoyl phosphatidylcholine (DOPC), dimyristoyl phosphatidylcholine (DMPC), distearoyl phosphatidylcholine (DSPC), 1-palmitoyl-2-stearoylphosphatidylcholine (PSPC), 1,2-diarachidoyl-sn-glycero-3-phosphatidylcholine (DPPC), 1-stearoyl-2-palmitoylphosphatidylcholine (SPPC), 1,2-dierucoyl-sn-glycero-3-phosphocholine (DEPC), palmitoyl oleoyl phosphatidylcholine (POPC), dilauroyl phosphatidylcholine (DLPC), palmitoyl stearoyl phosphatidylcholine (PSPC), lysophosphatidylcholine (LPC), dilinoleoyl phosphatidylcholine (DLPC), distearoyl phosphatidylethanolamine (DSPE), dimyristoyl phosphatidylethanolamine (DMPE), dipalmitoyl phosphatidylethanolamine (DPPE), dioleoyl phosphatidylethanolamine (dioleyl phosphatidylethanolamine, DOPE), palmitoyl oleoyl phosphatidylethanolamine (POPE), and sphingomyelin; preferably one or more selected from the group consisting of egg phosphatidylcholine (EPC), hydrogenated soybean phosphatidylcholine (HSPC), dioleoyl phosphatidylcholine (DOPC), and distearoyl phosphatidylcholine (DSPC); and more preferably one or both selected from the group consisting of egg phosphatidylcholine (EPC), and dioleoyl phosphatidylcholine (DOPC); and more preferably dioleoyl phosphatidylcholine (DOPC).

4.  The liposome composition according to any one of claims 1-3, wherein the sterol is one or more selected from the group consisting of cholesterol, 7-hydrocholesterol, lanosterol, sitosterol, brassicasterol, mycosterol, ostreasterol, stigmasterol, and ergosterol; preferably cholesterol.

5.  The liposome composition according to any one of claims 1-4, wherein the phospholipid is a zwitterionic phospholipid in combination with a pegylated phospholipid, an anionic phospholipid and/or a cationic phospholipid, the zwitterionic phospholipid is present in an amount of 50%-90%, the pegylated phospholipid is present in an amount of 0-40 wt%, the anionic phospholipid is present in an amount of 0-40 wt%, and the cationic phospholipid is present in an amount of 0-40 wt%, based on the total weight of the phospholipid.

6. The liposome composition according to any one of claims 1-5, wherein the mass ratio of Utidelone to the total phospholipid is in a range of from 0.2% to 30%; and/or, the mass ratio of the sterol to the total phospholipid is in a range of from 0% to 60%.

7. The liposome composition according to any one of claims 1-6, wherein the liposomes in the liposome composition have a particle size of from 50 nm to 250 nm; and/or
wherein the liposomes in the liposome composition have an average polydispersity index (PDI) of less than 0.25.

8. The liposome composition according to any one of claims 1-7, wherein the liposome composition is in a liquid form, or in a form of lyophilized powder.

9. The liposome composition according to claim 8, wherein the liposome composition further comprises or does not comprise a lyoprotectant; preferably, wherein the lyoprotectant is one or more selected from the group consisting of glucose, sucrose, maltose, lactose, mannose, trehalose, glycine, and dextran.

10. The liposome composition according to any one of claims 1-9, further comprising one or more of an osmoregulator, an antioxidant, a preservative, a pH regulator, and a buffer; preferably, the osmoregulator is one or more selected from the group consisting of sodium chloride, glycerin, sorbitol, mannitol, and glucose; preferably, the pH regulator is one or more selected from the group consisting of sodium hydroxide, sodium citrate, citric acid, phosphoric acid, acetic acid, and hydrochloric acid; preferably, the preservative is one or more selected from the group consisting of alkyl hydroxybenzoate, benzoic acid, sodium benzoate, sorbic acid, chlorhexidine acetate and benzalkonium bromide; preferably, the antioxidant is one or more selected from the group consisting of sodium sulfite, sodium bisulfite, sodium metabisulfite, sodium thiosulfate, ascorbic acid, *tert*-butyl *p*-hydroxyanisole, 2,6-di-*tert*-butylated hydroxytoluene and vitamin E; preferably, the buffer is one or more selected from the group consisting of citrate buffer, phosphate buffer, acetate buffer and Tris buffer.

11. The liposome composition according to any one of claims 1-10, obtained by one or more methods selected from the group consisting of a shear mixing method, a thin-film rehydration method, a spray drying method, a freeze drying method, a freeze-thaw method, a solvent injection method, a reverse phase evaporation method, an emulsification-evaporation method, a microfluidic method, an ultrasonication method, a supercritical fluid method, and a homogenization method.

12. A method for preparing the liposome composition according to any one of claims 1-11, which is a thin-film rehydration method, comprising the steps of:

   (1) mixing Utidelone, a phospholipid, and optionally a sterol with a solvent uniformly to obtain a mixed solution;
   (2) evaporating the mixed solution obtained in step (1) under reduced pressure to obtain a Utidelone-containing lipid film;
   (3) hydrating the lipid film obtained in step (2) to obtain a liposome solution;
   (4) subjecting the liposome solution obtained in step (3) to granule sizing to obtain a nanoliposome solution;
   (5) sterilizing the nanoliposome solution obtained in step (4); and
   (6) optionally, lyophilizing the drug-containing solution obtained in step (5).

13. The method according to claim 12, wherein the solvent used in step (1) is an organic solvent or a mixture of an organic solvent and water, preferably, the organic solvent is one or more selected from the group consisting of chloroform, dichloromethane, *tert*-butanol, isopropanol, ethyl acetate, ethanol, methanol, tetrahydrofuran, dioxane, acetonitrile, acetone, dimethyl sulfoxide, dimethylformamide, and methylpyrrolidone; and/or

   wherein the solution for the hydrating in step (3) is a solution comprising one or more of an osmoregulator, an antioxidant, a preservative, a pH regulator, and a buffer; and/or
   wherein the granule sizing in step (4) is carried out by using one or more methods selected from the group consisting of a shearing method, a high-pressure homogenization method and a microfluidization homogenization method.

**14.** The liposome composition according to any one of claims 1-11 for use in the prevention or treatment of a cancer.

**15.** A pharmaceutical formulation comprising the liposome composition according to any one of claims 1-11, and optionally an additional drug, preferably the additional drug is an anticancer drug, and preferably the pharmaceutical formulation is a pharmaceutical formulation for parenteral, inhalation, intraperitoneal, intravesical, intramuscular, intravenous, intratracheal, subcutaneous, intraocular, intrathecal, transdermal, rectal or intravaginal administraton, preferably the pharmaceutical formulation is a pharmaceutical formulation for intravenous administration; preferably the pharmaceutical formulation is a solid preparation, a liquid preparation, or a gas preparation, more preferably a liquid preparation for injection; more preferably, the pharmaceutical formulation is a stable aqueous suspension reconstituted from a sterile lyophilized powder of the liposome composition.

**Patentansprüche**

**1.** Liposomenzusammensetzung, umfassend Utidelon, ein Phospholipid und optional ein Sterol.

**2.** Liposomenzusammensetzung nach Anspruch 1, wobei das Phospholipid eines oder mehrere ausgewählt aus der Gruppe, bestehend aus einem pegylierten Phospholipid, einem anionischen Phospholipid, einem kationischen Phospholipid und einem zwitterionischen Phospholipid, ist oder wobei das Phospholipid eines oder mehrere ausgewählt aus der Gruppe, bestehend aus einem pegylierten Phospholipid, einem anionischen Phospholipid und einem zwitterionischen Phospholipid, ist; wobei das Phospholipid bevorzugt aus der Gruppe, bestehend aus einer Kombination eines pegylierten Phospholipids und eines zwitterionischen Phospholipids und einer Kombination eines anionischen Phospholipids und eines zwitterionischen Phospholipids, ausgewählt ist.

**3.** Liposomenzusammensetzung nach Anspruch 2, wobei das pegylierte Phospholipid eines oder mehrere ausgewählt aus der Gruppe, bestehend aus Distearoylphosphatidylethanolamin-Polyethylenglykol (DSPE-PEG), Distearoylphosphatidylethanolamin-Methoxypolyethylenglykol (DSPE-MPEG), Dimyristoylphosphatidylethanolamin-Polyethylenglykol (DMPE-PEG), Dipalmitoylglycerosuccinat-Polyethylenglykol (DPGS-PEG), cholesterylpegyliertem Phospholipid und ceramidopegyliertem Phospholipid, ist; bevorzugt ausgewählt aus der Gruppe, bestehend aus Distearoylphosphatidylethanolamin-Polyethylenglykol (DSPE-PEG) und Distearoylphosphatidylethanolamin-Methoxypolyethylenglykol (DSPE-MPEG); und eher bevorzugt Distearoylphosphatidylethanolamin-Methoxypolyethylenglykol (DSPE-MPEG); oder

wobei das anionische Phospholipid eines oder mehrere ausgewählt aus der Gruppe, bestehend aus Phosphatidylglycerin, Di(hexadecyl)phosphat (DhP), Phosphatidylinositol, Phosphatidylserin, Phosphatidylglycerin, Lysophosphatidylglycerin (Lysylphosphatidylglycerin, LPG), Phosphatidylethanolamin, Phosphatidsäure, Cardiolipin und Cholesterylhemisuccinat, ist; bevorzugt Phosphatidylglycerin; wobei das Phosphatidylglycerin bevorzugt eines oder mehrere ausgewählt aus der Gruppe, bestehend aus Dimyristoylphosphatidylglycerin (DMPG), Dipalmitoylphosphatidylglycerin (DPPG), Distearoylphosphatidylglycerin (DSPG), Dioleoylphosphatidylglycerin (DOPG), Dilauroylphosphatidylglycerin (DLPG), Eiphosphatidylglycerin (EPG), Eiphosphatidylinositol (EPI), Sojabohnenphosphatidylglycerin (SPG), Sojabohnenphosphatidylinositol (SPI), hydriertem Eiphosphatidylglycerin (HEPG), hydriertem Sojabohnenphosphatidylglycerin (HSPG), hydriertem Eiphosphatidylinositol (HEPI), Palmitoyl-Stearoyl-Phosphatidylglycerin (PSPG) und hydriertem Sojabohnenphosphatidylinositol (HSPI), ist; bevorzugt eines oder mehrere ausgewählt aus der Gruppe, bestehend aus Distearoylphosphatidylglycerin (DSPG), hydriertem Eiphosphatidylglycerin (HEPG) und hydriertem Sojabohnenphosphatidylglycerin (HSPG); und bevorzugt Distearoylphosphatidylglycerin (DSPG); oder
wobei das zwitterionische Phospholipid eines oder mehrere ausgewählt aus der Gruppe, bestehend aus Eiphosphatidylcholin (EPC), Eiphosphatidylserin (EPS), Phosphatidylethanolamin (EPE), Sojabohnenphosphatidylserin (SPS), Sojabohnenphosphatidylethanolamin (SPE), hydriertem Eiphosphatidylcholin (HEPC), hydriertem Eiphosphatidylserin (HEPS), hydriertem Eiphosphatidylethanolamin (HEPE), hydriertem Sojaphosphatidylcholin (HSPC), hydriertem Sojaphosphatidylserin (HSPS), hydriertem Sojaphosphatidylethanolamin (HSPE), Dipalmitoylphosphatidylcholin (DPPC), 1-Palmitoyl-2-Myristoylphosphatidylcholin (PMPC), 1-Myristoyl-2-Palmitoylphosphatidylcholin (MPPC), Dioleoylphosphatidylcholin (DOPC), Dimyristoylphosphatidylcholin (DMPC), Distearoylphosphatidylcholin (DSPC), 1-Palmitoyl-2-stearoylphosphatidylcholin (PSPC), 1,2-Diarachidoyl-sn-glycero-3-phosphatidylcholin (DBPC), 1-Stearoyl-2-palmitoylphosphatidylcholin (SPPC), 1,2-Dierucoyl-sn-glycero-3-phosphocholin (DEPC), Palmitoyl-Oleoylphosphatidylcholin (POPC), Dilauroylphosphatidylcholin (DLPC), Palmitoylstearoylphosphatidylcholin (PSPC), Lysophosphatidylcholin (LPC), Dilinoleoylphosphatidylcholin (DLPC), Distearoylphosphatidylethanolamin (DSPE), Dimyristoylphosphatidylethanolamin (DMPE), Dipalmitoylphosphatidylethanolamin (DPPE), Dioleoylphosphatidylethanolamin (Dioleylphosphatidylethanolamin, DOPE), Palmitoyl-Oleoylphosphatidylethanolamin (POPE) und Sphingomyelin, ist; bevorzugt eines oder mehrere aus der Gruppe ausgewählt, bestehend aus Eiphosphatidylcholin (EPC), hydriertem Sojabohnenphosphatidylcholin (HSPC), Dioleoylphosphatidylcholin (DOPC) und Distearoylphosphatidylcholin (DSPC); und eher bevorzugt eines oder beides ausgewählt aus der Gruppe, bestehend aus Eiphosphatidylcholin (EPC) und Dioleoylphosphatidylcholin (DOPC); und eher bevorzugt Dioleoylphosphatidylcholin (DOPC).

4. Liposomenzusammensetzung nach einem der Ansprüche 1 bis 3, wobei das Sterol eines oder mehrere ausgewählt aus der Gruppe, bestehend aus Cholesterin, 7-Hydrocholesterin, Lanosterol, Sitosterol, Brassicasterol, Mycosterol, Ostreasterol, Stigmasterol und Ergosterol, ist; bevorzugt Cholesterin.

5. Liposomenzusammensetzung nach einem der Ansprüche 1 bis 4, wobei das Phospholipid ein zwitterionisches Phospholipid in Kombination mit einem pegylierten Phospholipid, einem anionischen Phospholipid und/oder einem kationischen Phospholipid ist, wobei das zwitterionische Phospholipid, basierend auf dem Gesamtgewicht des Phospholipids, in einer Menge von 50 % bis 90 % vorhanden ist, das pegylierte Phospholipid in einer Menge von 0 bis 40 Gew.-% vorhanden ist, das anionische Phospholipid in einer Menge von 0 bis 40 Gew.-% vorhanden ist und das kationische Phospholipid in einer Menge von 0 bis 40 Gew.-% vorhanden ist.

6. Liposomenzusammensetzung nach einem der Ansprüche 1 bis 5, wobei das Massenverhältnis von Utidelon zu dem Gesamtphospholipid in einem Bereich von 0,2 % bis 30 % ist; und/oder das Massenverhältnis des Sterols zu dem Gesamtphospholipid in einem Bereich von 0 % bis 60 % ist.

7. Liposomenzusammensetzung nach einem der Ansprüche 1 bis 6, wobei die Liposomen in der Liposomenzusammensetzung eine Teilchengröße von 50 nm bis 250 nm aufweisen; und/oder wobei die Liposomen in der Liposomenzusammensetzung einen durchschnittlichen Polydispersitätsindex (PDI) von weniger als 0,25 aufweisen.

8. Liposomenzusammensetzung nach einem der Ansprüche 1 bis 7, wobei die Liposomenzusammensetzung in flüssiger Form oder in Form eines lyophilisierten Pulvers ist.

9. Liposomenzusammensetzung nach Anspruch 8, wobei die Liposomenzusammensetzung ferner ein Lyoprotektivum umfasst oder nicht umfasst; wobei das Lyoprotektivum bevorzugt eines oder mehrere ausgewählt aus der Gruppe, bestehend aus Glucose, Saccharose, Maltose, Lactose, Mannose, Trehalose, Glycin und Dextran, ist.

10. Liposomenzusammensetzung nach einem der Ansprüche 1 bis 9, ferner umfassend eines oder mehrere von einem Osmoregulator, einem Antioxidationsmittel, einem Konservierungsmittel, einem pH-Regulator und einem Puffer; wobei der Osmoregulator bevorzugt eines oder mehrere ausgewählt aus der Gruppe, bestehend aus Natriumchlorid, Glycerin, Sorbitol, Mannitol und Glucose, ist; wobei der pH-Regulator bevorzugt eines oder mehrere ausgewählt aus der Gruppe, bestehend aus Natriumhydroxid, Natriumzitrat, Zitronensäure, Phosphorsäure, Essigsäure und Salzsäure, ist; wobei das Konservierungsmittel bevorzugt eines oder mehrere ausgewählt aus der Gruppe, bestehend aus Alkylhydroxybenzoat, Benzoesäure, Natriumbenzoat, Sorbinsäure, Chlorhexidinacetat und Benzalkoniumbromid, ist; wobei das Antioxidationsmittel bevorzugt eines oder mehrere ausgewählt aus der Gruppe, bestehend aus Natriumsulfit, Natriumbisulfit, Natriummetabisulfit, Natriumthiosulfat, Ascorbinsäure, *tert*-Butyl-*p*-Hydroxyanisol, 2,6-di-*tert*-Butylhydroxytoluol und Vitamin E, ist; wobei der Puffer bevorzugt eines oder mehrere ausgewählt aus der Gruppe, bestehend aus Citratpuffer, Phosphatpuffer, Acetatpuffer und Tris-Puffer, ist.

11. Liposomenzusammensetzung nach einem der Ansprüche 1 bis 10, erlangt durch ein oder mehrere Verfahren, die aus der Gruppe, bestehend aus einem Schermischverfahren, einem Dünnfilm-Rehydratationsverfahren, einem Sprühtrocknungsverfahren, einem Gefriertrocknungsverfahren, einem Gefrierauftauverfahren, einem Lösungsmittelinjektionsverfahren, einem Umkehrphasenverdampfungsverfahren, einem Emulgierverdampfungsverfahren, einem Mikrofluidikverfahren, einem Ultraschallverfahren, einem Verfahren mit überkritischem Fluid und einem Homogenisierungsverfahren, ausgewählt sind.

12. Verfahren zum Herstellen der Liposomenzusammensetzung nach einem der Ansprüche 1 bis 11, das ein Dünnfilm-Rehydratationsverfahren ist, umfassend die folgenden Schritte:

(1) gleichmäßiges Mischen von Utidelon, einem Phospholipid und optional einem Sterol mit einem Lösungsmittel, um eine gemischte Lösung zu erlangen;
(2) Verdampfen der in Schritt (1) erlangten gemischten Lösung unter vermindertem Druck, um einen Utidelon enthaltenden Lipidfilm zu erlangen;

(3) Hydratisieren des in Schritt (2) erlangten Lipidfilms, um eine Liposomenlösung zu erlangen;

(4) Unterziehen der in Schritt (3) erlangten Liposomenlösung einem Korngrößenbestimmen, um eine Nanoliposomenlösung zu erlangen;

(5) Sterilisieren der in Schritt (4) erlangten Nanoliposomenlösung; und

(6) optionales Lyophilisieren der in Schritt (5) erlangten Arzneimittel enthaltenden Lösung.

13. Verfahren nach Anspruch 12, wobei das in Schritt (1) verwendete Lösungsmittel ein organisches Lösungsmittel oder ein Gemisch aus einem organischen Lösungsmittel und Wasser ist, wobei das organische Lösungsmittel bevorzugt eines oder mehrere aus der Gruppe, bestehend aus Chloroform, Dichlormethan, tert-Butanol, Isopropanol, Ethylacetat, Ethanol, Methanol, Tetrahydrofuran, Dioxan, Acetonitril, Aceton, Dimethylsulfoxid, Dimethylformamid und Methylpyrrolidon, ist; und/oder

wobei die Lösung für das Hydratisieren in Schritt (3) eine Lösung ist, umfassend einen oder mehrere Osmoregulatoren, ein Antioxidans, ein Konservierungsmittel, einen pH-Regulator und einen Puffer; und/oder
wobei das Korngrößenbestimmen in Schritt (4) mithilfe eines oder mehrerer Verfahren durchgeführt wird, das aus der Gruppe, bestehend aus einem Scherverfahren, einem Hochdruckhomogenisierungsverfahren und einem Mikrofluidisierungshomogenisierungsverfahren, ausgewählt ist.

14. Liposomenzusammensetzung nach einem der Ansprüche 1 bis 11 zur Verwendung bei der Prävention oder Behandlung von Krebs.

15. Pharmazeutische Formulierung, umfassend die Liposomenzusammensetzung nach einem der Ansprüche 1 bis 11 und optional ein zusätzliches Arzneimittel, wobei das zusätzliche Arzneimittel bevorzugt ein Krebsmedikament ist und wobei die pharmazeutische Formulierung bevorzugt eine pharmazeutische Formulierung zur parenteralen, inhalativen, intraperitonealen, intravesikalen, intramuskulären, intravenösen, intratrachealen, subkutanen, intraokularen, intrathekalen, transdermalen, rektalen oder intravaginalen Verabreichung ist, wobei die pharmazeutische Formulierung bevorzugt eine pharmazeutische Formulierung zur intravenösen Verabreichung ist; wobei ist die pharmazeutische Formulierung bevorzugt ein festes Präparat, ein flüssiges Präparat oder ein Gaspräparat, eher bevorzugt ein flüssiges Präparat zur Injektion ist; wobei die pharmazeutische Formulierung eher bevorzugt eine stabile wässrige Aufschwemmung ist, die aus einem sterilen lyophilisierten Pulver der Liposomenzusammensetzung rekonstituiert wurde.

## Revendications

1. Composition de liposomes comprenant de l'Utidelone, un phospholipide et éventuellement un stérol.

2. Composition de liposomes selon la revendication 1, dans laquelle le phospholipide est un ou plusieurs composés choisis dans le groupe constitué par un phospholipide pégylé, un phospholipide anionique, un phospholipide cationique et un phospholipide zwittérionique, ou dans laquelle le phospholipide est un ou plusieurs composés choisis dans le groupe constitué par un phospholipide pégylé, un phospholipide anionique et un phospholipide zwittérionique ; de préférence, le phospholipide est choisi dans le groupe constitué par une combinaison d'un phospholipide pégylé et d'un phospholipide zwittérionique, et une combinaison d'un phospholipide anionique et d'un phospholipide zwittérionique.

3. Composition de liposomes selon la revendication 2, dans laquelle le phospholipide pégylé est un ou plusieurs composés choisis dans le groupe constitué par le distéaroyl phosphatidyléthanolamine-polyéthylène glycol (DSPE-PEG), le distéaroyl phosphatidyléthanolamine-méthoxypolyéthylène glycol (DSPE-MPEG), le dimyristoyl phosphatidyléthanolamine-polyéthylène glycol (DMPE-PEG), le glycérosuccinate de dipalmitoyle-polyéthylène glycol (DPGS-PEG), le cholestéryle-phospholipide pégylé et le céramido-phospholipide pégylé ; de préférence choisi dans le groupe constitué par le distéaroyl phosphatidyléthanolamine-polyéthylène glycol (DSPE-PEG) et le distéaroyl phosphatidyléthanolamine-méthoxypolyéthylène glycol (DSPE-MPEG) ; et plus particulièrement le distéaroyl phosphatidyléthanolamine-méthoxypolyéthylène glycol (DSPE-MPEG) ; ou

dans laquelle le phospholipide anionique est un ou plusieurs composés choisis dans le groupe constitué par le phosphatidylglycérol, le di(hexadécyl)phosphate (DhP), le phosphatidylinositol, la phosphatidylsérine, le phosphatidylglycérol, le lysophosphatidylglycérol (lysylphosphatidylglycérol, LPG), la phosphatidyléthanolamine, l'acide phosphatidique, la cardiolipine et l'hémisuccinate de cholestérol ; de préférence le phosphatidylglycérol ; de préférence, le phosphatidylglycérol est un ou plusieurs composés choisis dans le groupe constitué par le dimyristoyl phosphatidylglycérol (DMPG), le dipalmitoyl phosphatidylglycérol (DPPG), le distéaroyl phosphatidylglycérol (DSPG), le dioléoyl phosphatidylglycérol (DOPG), le dilauroyl phosphatidylglycérol (DLPG), le phosphatidylglycérol d'œuf (EPG), le phosphatidylinositol d'œuf (EPI), le phosphatidylglycérol de soja (SPG), le phosphatidylinositol de soja (SPI), le phosphatidylglycérol d'œuf hydrogéné (HEPG), le phosphatidylglycérol de soja hydrogéné (HSPG), le phosphatidylinositol d'œuf hydrogéné (HEPI), le palmitoyl stéaroyl phosphatidylglycérol (PSPG) et le phosphatidylinositol de soja (HSPI) ; de préférence un ou plusieurs composés choisis dans le groupe constitué par le distéaroyl phosphatidylglycérol (DSPG), le phosphatidylglycérol d'œuf hydrogéné (HEPG) et le phosphatidylglycérol de soja hydrogéné (HSPG) ; et de préférence le distéaroyl phosphatidylglycérol (DSPG) ; ou

dans laquelle le phospholipide zwitterionique est un ou plusieurs composé choisis dans le groupe constitué par la phosphatidylcholine d'œuf (EPC), la phosphatidylsérine d'œuf (EPS), la phosphatidyléthanolamine (EPE), la phosphatidylsérine de soja (SPS), la phosphatidyléthanolamine de soja (SPE), la phosphatidylcholine d'œuf hydrogénée (HEPC), la phosphatidylsérine d'œuf hydrogénée (HEPS), la phosphatidyléthanolamine d'œuf hydrogénée (HEPE), la phosphatidylcholine de soja hydrogénée (HSPC), la phosphatidylsérine de soja hydrogénée (HSPS), la phosphatidyléthanolamine de soja hydrogénée (HSPE), la dipalmitoyl phosphatidylcholine (DPPC), la 1-palmitoyl-2-myristoylphosphatidylcholine (PMPC), la 1-myristoyl-2-palmitoylphosphatidylcholine (MPPC), la dioléoyl phosphatidylcholine (DOPC), la dimyristoyl phosphatidylcholine (DMPC), la distéaroyl phosphatidylcholine (DSPC), la 1-palmitoyl-2-stéaroylphosphatidylcholine (PSPC), la 1,2-diarachidoyl-sn-glycéro-3-phosphatidylcholine (DBPC), la 1-stéaroyl-2-palmitoylphosphatidylcholine (SPPC), la 1,2-diérucoyl-sn-glycéro-3-phosphocholine (DEPC), la palmitoyl oléoyl phosphatidylcholine (POPC), la dilauroyl phosphatidylcholine (DLPC), la palmitoyl stéaroyl phosphatidylcholine (PSPC), la lysophosphatidylcholine (LPC), la dilinoléoyl phosphatidylcholine (DLPC), la distéaroyl phosphatidyléthanolamine (DSPE), la dimyristoyl phosphatidyléthanolamine (DMPE), la dipalmitoyl phosphatidyléthanolamine (DPPE), la dioléoyl phosphatidyléthanolamine (dioleyl phosphatidyléthanolamine, DOPE), la palmitoyl oléoyl phosphatidyléthanolamine (POPE) et la sphingomyéline ; de préférence un ou plusieurs composés choisis dans le groupe constitué par la phosphatidylcholine d'œuf (EPC), la phosphatidylcholine de soja hydrogénée (HSPC), la dioléoyl phosphatidylcholine (DOPC) et la distéaroyl phosphatidylcholine (DSPC) ; et plus préférablement un ou les deux composés choisis dans le groupe constitué par la phosphatidylcholine d'œuf (EPC) et la dioléoyl phosphatidylcholine (DOPC) ; et plus préférablement la dioléoyl phosphatidylcholine (DOPC).

4. Composition de liposomes selon l'une quelconque des revendications 1 à 3, dans laquelle le stérol est un ou plusieurs composés choisis dans le groupe constitué par le cholestérol, le 7-hydrocholestérol, le lanostérol, le sitostérol, le brassicastérol, le mycostérol, l'ostréastérol, le stigmastérol et l'ergostérol ; de préférence le cholestérol.

5. Composition de liposomes selon l'une quelconque des revendications 1 à 4, dans laquelle le phospholipide est un phospholipide zwitterionique en combinaison avec un phospholipide pégylé, un phospholipide anionique et/ou un phospholipide cationique, le phospholipide zwitterionique est présent en une quantité de 50 % à 90 %, le phospholipide pégylé est présent en une quantité de 0 à 40 % en poids, le phospholipide anionique est présent en une quantité de 0 à 40 % en poids, et le phospholipide cationique est présent en une quantité de 0 à 40 % en poids, par rapport au poids total du phospholipide.

6. Composition de liposomes selon l'une quelconque des revendications 1 à 5, dans laquelle le rapport massique de l'Utidelone au phospholipide total est dans une plage de 0,2 % à 30 % ; et/ou le rapport massique du stérol au phospholipide total est dans une plage de 0 % à 60 %.

7. Composition de liposomes selon l'une quelconque des revendications 1 à 6, dans laquelle les liposomes dans la composition de liposomes ont une taille de particules de 50 nm à 250 nm ; et/ou
dans laquelle les liposomes dans la composition de liposomes ont un indice de polydispersité moyen (PDI) inférieur à 0,25.

8. Composition de liposomes selon l'une quelconque des revendications 1 à 7, dans laquelle la composition de liposomes est sous forme liquide ou sous forme de poudre lyophilisée.

9. Composition de liposomes selon la revendication 8, dans laquelle la composition de liposomes comprend également ou ne comprend pas un lyoprotecteur ; de préférence, dans laquelle le lyoprotecteur est un ou plusieurs composés choisis dans le groupe constitué par le glucose, le saccharose, le maltose, le lactose, le mannose, le tréhalose, la glycine et le dextrane.

10. Composition de liposomes selon l'une quelconque des revendications 1 à 9, comprenant également un ou plusieurs composés parmi un osmorégulateur, un antioxydant, un conservateur, un régulateur de pH, et un tampon ; de préférence, l'osmorégulateur est un ou plusieurs composés choisis dans le groupe constitué par le chlorure de sodium, la glycérine, le sorbitol, le mannitol et le glucose ; de préférence, le régulateur de pH est un ou plusieurs composés choisis dans le groupe constitué par l'hydroxyde de sodium, le citrate de sodium, l'acide citrique, l'acide phosphorique, l'acide acétique et l'acide chlorhydrique ; de préférence, le conservateur est un ou plusieurs composés choisis dans le groupe constitué par l'hydroxybenzoate d'alkyle, l'acide benzoïque, le benzoate de sodium, l'acide sorbique, l'acétate de chlorhexidine et le bromure de benzalkonium ; de préférence, l'antioxydant est un ou plusieurs composés choisis dans le groupe constitué par le sulfite de sodium, le bisulfite de sodium, le métabisulfite de sodium, le thiosulfate de sodium, l'acide ascorbique, le *tert*-butyl-*p*-hydroxyanisole, l'hydroxytoluène 2,6-di-*tert*-butylé et la vitamine E ; de préférence, le tampon est un ou plusieurs composés choisis dans le groupe constitué par le tampon citrate, le tampon phosphate, le tampon acétate et le tampon Tris.

11. Composition de liposomes selon l'une quelconque des revendications 1 à 10, obtenue par un ou plusieurs procédés choisis dans le groupe constitué par un procédé de mélange par cisaillement, un procédé de réhydratation en couche mince, un procédé de séchage par atomisation, un procédé de lyophilisation, un procédé de congélation-décongélation, un procédé d'injection de solvant, un procédé d'évaporation en phase inverse, un procédé d'émulsification-évaporation, un procédé microfluidique, un procédé d'ultrasonication, un procédé de fluide supercritique et un procédé d'homogénéisation.

12. Procédé de préparation de la composition de liposomes selon l'une quelconque des revendications 1 à 11, qui est un procédé de réhydratation en film mince, comprenant les étapes de :

(1) mélange uniforme de l'Utidelone, d'un phospholipide et éventuellement d'un stérol avec un solvant pour obtenir une solution mixte ;
(2) évaporation de la solution mixte obtenue à l'étape (1) sous pression réduite pour obtenir un film lipidique contenant de l'Utidelone ;
(3) hydratation du film lipidique obtenu à l'étape (2) pour obtenir une solution de liposomes ;
(4) soumission de la solution de liposomes obtenue à l'étape (3) à un calibrage des granulés pour obtenir une solution de nanoliposomes ;
(5) stérilisation de la solution de nanoliposomes obtenue à l'étape (4) ; et
(6) éventuellement, lyophilisation de la solution contenant le médicament obtenue à l'étape (5).

13. Procédé selon la revendication 12, dans lequel le solvant utilisé à l'étape (1) est un solvant organique ou un mélange d'un solvant organique et d'eau, de préférence, le solvant organique est un ou plusieurs solvants choisis dans le groupe constitué par le chloroforme, le dichlorométhane, *le tert*-butanol, l'isopropanol, l'acétate d'éthyle, l'éthanol, le méthanol, le tétrahydrofurane, le dioxane, l'acétonitrile, l'acétone, le diméthylsulfoxyde, le diméthylformamide et la méthylpyrrolidone et/ou

dans laquelle la solution pour l'hydratation à l'étape (3) est une solution comprenant un ou plusieurs composés parmi un osmorégulateur, un antioxydant, un conservateur, un régulateur de pH et un tampon ; et/ou
dans lequel le calibrage des granulés à l'étape (4) est effectué en utilisant un ou plusieurs procédés choisis dans le groupe constitué par un procédé de cisaillement, un procédé d'homogénéisation à haute pression et un procédé d'homogénéisation par microfluidisation.

14. Composition de liposomes selon l'une quelconque des revendications 1 à 11 destinée à être utilisée dans la prévention ou le traitement d'un cancer.

15. Formulation pharmaceutique comprenant la composition de liposomes selon l'une quelconque des revendications 1 à 11, et éventuellement un médicament supplémentaire, de préférence le médicament supplémentaire est un médicament anticancéreux, et de préférence la formulation pharmaceutique est une formulation pharmaceutique pour administration parentérale, par inhalation, intrapéritonéale, intravésicale, intramusculaire, intraveineuse, intratrachéale, sous-cutanée, intraoculaire, intrathécale, transdermique, rectale ou intravaginale, de préférence la formulation pharmaceutique est une formulation pharmaceutique pour administration intraveineuse ; de préférence la formulation pharmaceutique est une préparation solide, une préparation liquide ou une préparation gazeuse, plus préférablement une préparation liquide pour injection ; plus préférablement, la formulation pharmaceutique est une suspension aqueuse stable reconstituée à partir d'une poudre lyophilisée stérile de la composition de liposomes.

Size Distribution by Intensity

FIG.1

FIG.2

FIG.3

FIG.4

FIG.5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20090191264 A
- CN 100409846 C
- EP 2286795 A1